# EUROPEAN PATENT SPECIFICATION

(11) **EP 2 510 980 B1**
(45) Date of publication and mention of the grant of the patent: **08.06.2016**
(21) Application number: 12161209.7
(22) Date of filing: 22.10.2009
(51) Int. Cl.: A61Q 7/00, A61Q 19/08, A61Q 19/10, A61K 8/64, A61K 8/02

(54) **A method for the dermocosmetic treatment of skin by application of NGF-containing compositions**
Verfahren für eine dermokosmetische Behandlung der Haut mittels NGF-enthaltende Zusammensetzungen
Méthode de traitement dermocosmétique de la peau par application de compositions comprenant un NGF

(30) Priority: 23.10.2008 IT MI20081877
(43) Date of publication of application: 17.10.2012
(62) Divisional of application: 09173799.9
(73) Proprietor: De Carli, Edda, 37069 Villafranca di Verona (VR) (IT)
(72) Inventor: De Carli, Edda, 37069 Villafranca di Verona (VR) (IT); Brunetta, Fabio, 31041 Cornuda (TV) (IT)
(74) Representative: Ferreccio, Rinaldo

(56) References cited:
- EP-A- 1 262 169
- WO-A2-2007/070850
- JP-A- 11 029 441
- JP-A- 11 255 623
- US-A- 4 673 526
- US-A1- 2003 161 870
- US-A1- 2005 153 873
- US-B1- 6 867 179

## Description

### Field of the invention

The present invention relates to the field of the cosmetics industry and in particular refers to methods for the dermocosmetic treatment of human skin which provide the application of compositions containing nerve cell growth factor (NGF).

### Background of the invention

Immunohistochemical studies have demonstrated that receptors for nerve cell growth factor (NGF), namely the low-affinity receptor p75 and the high-affinity receptor TrKA, are expressed on the basal keratinocytes of normal human skin. Messenger RNAs from the high- and low-affinity receptors for NGF have also been detected in cultured human keratinocytes (Pincelli C. et al, "Expression and function of nerve growth factor receptor on cultured keratinocytes", J. Invest. Dermatol. 1994, 103: 13-8).

NGF is capable of inducing the proliferation of keratinocytes to a greater extent than another growth factor present in the skin, namely epidermal growth factor (EGF).

Moreover, the skin is a tissue which is located at the boundary between the body and the external environment and is a peripheral zone characterized by extraordinary reactive potential due to the existence of a neuro-immuno-cutaneous system based on the morpho-functional interaction between the nervous system, the immune system and the skin.

Within the skin, there are huge numbers of ultrastructural contacts between nerve fibres, dermal and epidermal cells and immune cells and, among these, the first are capable of locally releasing numerous neuromediators, many of which are also secreted by dermal/epidermal cells.

The fact that skin cells (not just the keratinocytes but also other cells, including Langerhans cells, mastocytes, the endothelial cells of dermal microvessels) express NGF receptors creates the basis for a delicate network through which the peripheral nervous system controls extremely important cutaneous processes, such as cellular proliferation and differentiation, cytokine production and antigen presentation.

It has furthermore been shown that NGF favors tissue repair by means of the proliferation of resident cells, such as for example keratinocytes. It has more recently been shown that keratinocytes originating from diabetic individuals exhibit an NGF content which is very much lower than normal, an abnormality which may be associated not only with dysfunctional cutaneous sensory fibres but also with the healing difficulties typical of diabetes.

Fibroblasts, which are particularly abundant in the dermis, produce and release considerable amounts of NGF, which exerts its own effects in the keratinocytes, or on dermal immune cells such as macrophages, in which NGF stimulates phagocytosis, receptor expression and the production and release of cytokines such as IL-1β.

With advancing age and, for women, following the menopause, a reduction in cutaneous innervation is observed, the consequences of which include a reduction in the input of neuromediators and growth factors, in particular NGF, into the skin, which results in a reduction in epidermal cell proliferation, with consequent thinning of the skin, which takes on an ever drier and rough appearance.

To overcome this, patent application EP 1 262 169 proposed the topical application of a composition comprising at least one substance which has an activity on cutaneous nerve trophicity and/or a neurostimulant and/or a neuroactivator and/or a neuromediator. In particular, NGF was suggested as a substance having an activity on cutaneous nerve trophicity.

In reality none of the examples shown in the patent application in question relates to a composition containing NGF, but instead to compositions containing capsaicin, capsicum extracts, PEG or hyaluronic acid. This is probably due to the fact that the absorption and bioavailability of NGF after applying conventional cosmetic compositions to the skin are extremely low if not zero.

The use of NGF for topical application has also been suggested in patents US 5 427 778 and US 6 063 757, but restricted to the treatment of skin or corneal wounds by means of gel formulations which contain it.

Tropical application of NGF, in particular of βNGF obtained from recombinant insect cells containing a certain recombinant Baculovirus, is also suggested in patent application EP 0 444 638, but restricted to therapeutic use.

Similarly, application WO 2007/034266 mentions pharmaceutical compositions for topical application in the case of skin or corneal ulcers, containing human recombinant NGF (beta rhNGF) but no mention is made of using them for cosmetic purposes.

WO 2007/070850 discloses a skin care composition containing a skin care agent, a carrier and a penetration agent chosen from follicular penetration enhancers, chemical penetration enhancers and active penetration enhancers; to further enhance skin penetration additional techniques like iontophoresis, ultrasound, electroporation, tape stripping, gene guns, tines or microneedles are listed; no citation of NGF is present in the document.

US 2005/0153873 describes a delivery system for the transdermal delivery of a biologically active agent to a subject, comprising a microprojection member having a plurality of stratum corneum-piercing microprojections, a formulation containing a biologically active agent and an oscillation inducing device cooperating with the microprojection members to produce high frequency oscillations. No tests of efficacy of are present in this document which names hundreds of possible active agents, without mentioning NGF.

US 2003/0161870 discloses a penetration enhancer composition for increasing skin permeability to topically or transdermally administer active agents and indicates electrophoresis, e.g. iontophoresis as a physical means to increase skin penetration.

### Brief description of the invention

The problem underlying the present invention was that of providing a dermocosmetic method for reducing the effects of skin ageing, for regenerating stretch marks (striae), scars, by means of an increased input of NGF into skin.

The word "skin" is intended to denote the skin of the whole body (torso, upper limbs, lower limbs, external mucous membranes), including the scalp.

With particular reference to the scalp, one of the objects of the invention is also that of providing a cosmetic method for reinvigorating the hair bulbs and favoring hair growth.

According to one aspect of the present invention, the above-started technical problem has been solved by a method comprising the steps of:
a) providing a composition containing a cosmetically effective amount of NGF;
b) applying the above-stated composition onto skin areas in need of cosmetic treatment, bringing about the absorption of NGF into the skin by applying appropriate means suitable for favoring penetration of NGF into the skin, selected from the group consisting of tools suitable for bringing about scarification of the epidermis, which are applied before applying the composition containing NGF, and tattoo needles or tattoo needle machines.

The tools suitable for bringing about scarification of the epidermis may comprise, for example, sterile needles, curettes.

The NGF usable in the method according to the invention may be of either murine or recombinant human origin, the latter being preferred.

The NGF is contained in the cosmetic composition used in the method according to the present invention in amounts comprised between 10⁻⁸% and 5% by weight of the total weight of the composition, preferably in an amount comprised between 10⁻⁵% and 0.5% by weight of the total weight of the composition.

The cosmetic composition used in the method according to the present invention contains a cosmetically acceptable vehicle and may contain further cosmetically active substances, such as for example vitamins, essential fatty acids, ceramides, proteins or protein hydrolysates, amino acids, polyols, plant extracts and hydroxy acids, antioxidants, retinol, retinol palmitate, plant meristematic and stem cells.

Among the vitamins, vitamin E, vitamin C, vitamin PP and other group B vitamins and the derivatives thereof may in particular be mentioned.

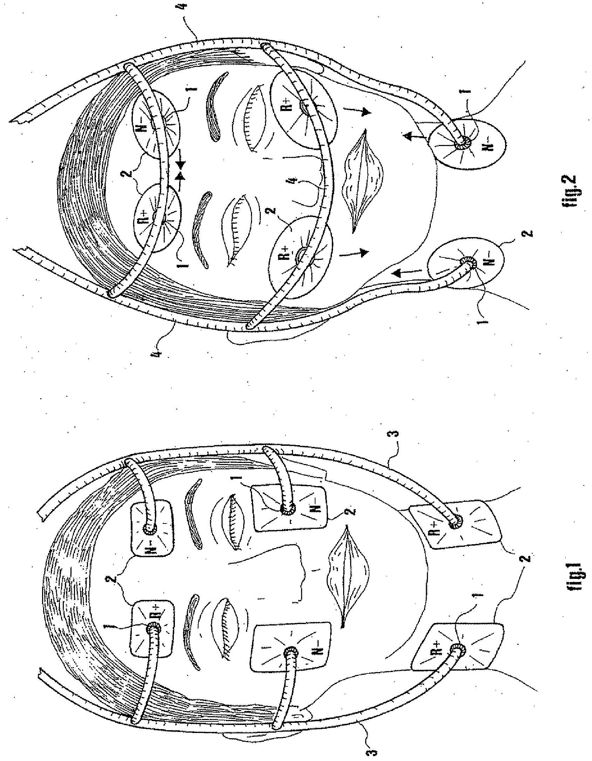

A device which may conveniently be used for transcutaneous ionophoresis is the device shown in the present Applicant's patent IT 1 315 196, which provides the use of a mesh of microelectrodes borne on electrically conductive rubber or silicone rubber disks, provided with ionophoresis sponge, which are placed on the areas of skin to be treated with an interposed moist pad, for example of gauze, as shown in the appended Figures 1 and 2.

### Detailed description of the invention

The present invention will now be further illustrated with reference to some examples which will be described below in a purely illustrative, nonlimiting manner.

### EXAMPLE 1

According to one embodiment of the method according to the present invention, a cosmetic composition is provided containing, in weight/weight percentages:
Freeze-dried NGF-7S (Sigma-Aldrich) 0.0008 Physiological saline buffered to pH 7.2
with 0.15 M phosphate buffer q.s. to 100

The above-described solution is applied to the dermis of the skin or to the scalp by means of a tattoo needle machine. In general, a volume of 0.1 ml per cm² of skin is introduced by means of the needles, or using a linear technique for deep wrinkles.

### EXAMPLE 2 (not in accordance with the invention)

| | |
|---|---|
| Acrylic crosspolymer (Carbopol Ultrez 21, Noveon) | 0.6 |
| Sodium hydroxide solution | q.s. to pH 7 |
| Freeze-dried NGF-7S (Sigma-Aldrich) | 0.0001 |
| NaCl | 0.6 |
| Methyl parahydroxybenzoate | 0.15 |
| Sterile purified water | q.s. to 100 |

Using the above-listed components, a hydrophilic transparent gel with a viscosity at 10 rpm and 25°C of 3100 mPa·s (Brookfield RTV DVII viscosimeter, small sample adapter, impeller no. 29) was prepared in conventional manner.

The area of skin to be treated is first of all (after cleansing) subjected to deep peeling by applying an acidic composition for a sufficient time to achieve softening of the horny layer. The skin is then preferably rinsed with water or swabbed with an alkaline solution in such a manner as to adjust the pH towards neutrality, after which the above-described gel is spread uniformly, in accordance with the polarity corresponding to the electrode, on the skin previously subjected to peeling and then a mesh of six microelectrodes in silicone rubber, provided with ionophoresis sponge, as shown in Fig. 1, is applied with an interposed piece of moist disposable gauze. A conveniently calibrated voltage (for example 0.5 mA/cm²) is then applied using a medium frequency unidirectional current, for the time necessary to ensure that the ionophoretic treatment permits transcutaneous penetration of the composition and absorption of the active substance. This time is generally between 20 and 30 minutes.

A "FIRING" model ionophoresis apparatus from "COSMOGAMMA by emildue" was used.

### EXAMPLE 3

| | |
|---|---|
| Purified lecithin (Phospholipon 80 Natterman) | 2.0 |
| Freeze-dried NGF-7S (Sigma-Aldrich) | 0.001 |
| Cholesterol, USP | 1.0 |
| Citrate buffer | q.s. to pH 7 |
| Methyl parahydroxybenzoate | 0.15 |
| Sterile purified water | q.s. to 100 |

The values shown above are parts by weight per hundred parts by weight of the overall formulation.

Using the above-listed ingredients, a liposomal suspension was prepared by dispersing the cholesterol and the lecithin in an equal amount of ethanol and pouring the resultant dispersion into water at 90°C with a running turbine. After evaporating the ethanol (by means of a vacuum pump), the temperature is reduced to 50°C. The methyl parahydroxybenzoate and the citrate buffer are added. The temperature is further reduced to 5°C, after which the freeze-dried NGF is dispersed.

Before applying the liposomal suspension, the area of skin to be treated was gently scarified by means of sterilized needles, after which the suspension was spread uniformly with gentle rubbing to favor absorption thereof.

### EXAMPLE 4 (not in accordance with the invention)

| | |
|---|---|
| Xanthan gum (Rodicolare T, Rhodia) | 1.0 |
| Freeze-dried NGF | 0.0005 |
| NaCl | 0.6 |
| Methyl parahydroxybenzoate | 0.15 |
| 0.15 µM phosphate buffer | q.s. to pH 7 |
| Sterile purified water | q.s. to 100 |

The gel in question is applied using the same method as for the gel according to Example 2.

### EXAMPLE 5

| | |
|---|---|
| Freeze-dried NGF | 0.01 |
| Hydrogenated castor oil (Cutina HR Cognis) | 10.0 |
| Octyl palmitate | q.s. to 100 |

Using the above-stated ingredients, a thixotropic, lipophilic gel was prepared, the viscosity of which can be modified by varying the concentration of hydrogenated castor oil. Preparation is carried out by heating the octyl palmitate to 85°C, then dissolving the hydrogenated castor oil until a transparent oil is obtained and then cooling with stirring. Once cold, the NGF is then dispersed with stirring.

### EXAMPLE 6

| | |
|---|---|
| Freeze-dried NGF | 0.001 |
| Cyclopentasiloxane | q.s. to 100 |

The NGF is dispersed cold, possibly by means of a Silverson emulsifier. This preparation makes it possible to apply NGF suspended in a volatile silicone oil onto the skin. Once the silicone has evaporated, which takes approx. 30 minutes, pure NGF remains on the surface of the skin.

The effect of topical application of NGF in accordance with the method of the present invention may be confirmed for example using in vitro cell culture systems. It is, for example, possible to use an organotypic raft culture system, which permits the study of epidermal growth control (proliferation and differentiation of the keratinocytes) under conditions highly similar to those in vivo, such as that described by Steude J. et al., The Journal of Investigative Dermatology, Vol. 119, No. 6, December 2002, pp. 1254-1260.

## Claims

1. A method for the non-therapeutic, dermocosmetic treatment of skin, comprising the steps of:
- providing a composition containing a cosmetically effective amount of NGF of 10⁻⁸% to 5% by weight of the total weight of the composition;
- applying said composition onto skin areas in need of cosmetic treatment, bringing about the absorption of NGF into the skin by applying means suitable for favoring penetration of NGF into the skin, selected from the group consisting of tools suitable for bringing about scarification of the epidermis, which are applied before applying said composition, and tattoo needles or tattoo needle machines.

2. The method according to claim 1, wherein said tools are sterile needles or curettes.

3. The method according to any one of the preceding claims, wherein the NGF is human recombinant NGF.

4. The method according to any one of the preceding claim, wherein the NGF is contained in said composition in amounts comprised between 10-⁵% and 0.5% of the total weight of the composition.

5. The method according to any one of the preceding claims, wherein said cosmetic composition comprises a cosmetically acceptable vehicle and optionally further cosmetically active substances, selected from the group comprising vitamins, essential fatty acids, ceramides, proteins or protein hydrolysates, amino acids, polyols, plant extracts and hydroxy acids, antioxidants, retinol and retinol palmitate, plant meristematic and stem cells.

## Patentansprüche

1. Verfahren für die nicht therapeutische, dermokosmetische Behandlung von Haut, das folgende Schritte umfasst:
- Bereitstellen einer Zusammensetzung, die eine kosmetisch wirksame Menge an NGF von 10⁻⁸ bis 5 Gewichts-% des Gesamtgewichts der Zusammensetzung enthält;
- Auftragen der Zusammensetzung auf Hautbereiche, die einer kosmetischen Behandlung bedürfen, Bewirken der Absorption von NGF in die Haut durch ein Anwenden von Mitteln, die zum Begünstigen eines Eindringens von NGF in die Haut geeignet sind, ausgewählt aus der Gruppe bestehend aus Werkzeugen, die zum Bewirken einer Skarifizierung der Epidermis geeignet sind, die angewendet werden bevor die Zusammensetzung aufgebracht und Tätowierungsnadeln oder Tätowierungsnadelmaschinen angewendet werden.

2. Verfahren nach Anspruch 1, wobei die Werkzeuge sterile Nadeln oder Küretten sind.

3. Verfahren nach einem der vorhergehenden Ansprüche, wobei der NGF humaner rekombinanter NGF ist.

4. Verfahren nach einem der vorhergehenden Ansprüche, wobei der NGF in der Zusammensetzung in Mengen zwischen 10⁻⁵ und 0,5 % des Gesamtgewichts der Zusammensetzung enthalten ist.

5. Verfahren nach einem der vorhergehenden Ansprüche, wobei die kosmetische Zusammensetzung einen kosmetisch akzeptablen Träger und optional weitere kosmetisch wirksame Substanzen umfasst, ausgewählt aus der Gruppe umfassend Vitamine, essentielle Fettsäuren, Ceramide, Eiweiße oder Eiweißhydrolysate, Aminosäuren, Polyole, Pflanzenextrakte und Hydroxysäuren, Antioxidationsmittel, Retinol und Retinolpalmitat, pflanzliche Meristem- und Stammzellen.

## Revendications

1. Méthode pour le traitement non thérapeutique, dermo-cosmétique de la peau, comprenant les étapes de :
- fournir une composition contenant une quantité cosmétiquement efficace de NGF entre 10⁻⁸ et 5% en poids du poids total de la composition,
- appliquer la dite composition sur les zones de peau ayant besoin d'un traitement cosmétique, et provoquer l'absorption du NGF dans la peau par l'application d'un moyen approprié à favoriser la pénétration du NGF dans la peau, choisi dans le groupe composé d'outils appropriés à provoquer la scarification de l'épiderme, qui sont appliqués avant d'appliquer la dite composition, et les aiguilles de tatouage ou les machines à aiguilles de tatouage.

2. Méthode selon la revendication 1, **caractérisée en ce que** les dits outils sont des aiguilles ou des curettes stériles.

3. Méthode selon l'une quelconque des revendications précédentes, **caractérisée en ce que** le NGF est un NGF humain recombinant.

4. Méthode selon l'une quelconque des revendications précédentes, **caractérisé en ce que** le NGF est contenu dans la dite composition selon des quantités comprises entre 10⁻⁵ et 0,5% du poids total de la composition.

5. Méthode selon l'une quelconque des revendications précédentes, **caractérisée en ce que** la dite composition cosmétique comprend un véhicule cosmétiquement acceptable et éventuellement des substances cosmétiquement actives supplémentaires choisies dans le groupe comprenant les vitamines, les acides gras essentiels, les céramides, les protéines ou hydrolysats de protéine, les acides aminés, les polyols, les extraits de plantes et les acides hydroxyles, les antioxydants, le rétinol et le palmitate de rétinol, les cellules stématiques et méristématiques de plante.
